(19) Europäisches Patentamt
European Patent Office
Office européen des brevets

(11) **EP 4 194 041 A1**

(12) **EUROPEAN PATENT APPLICATION**

(43) Date of publication:
**14.06.2023 Bulletin 2023/24**

(21) Application number: **22212076.8**

(22) Date of filing: **07.12.2022**

(51) International Patent Classification (IPC):
**A61M 16/10** (2006.01)   **A61M 16/20** (2006.01)
**A61M 16/22** (2006.01)   **A61M 16/08** (2006.01)
**A61M 16/00** (2006.01)

(52) Cooperative Patent Classification (CPC):
**A61M 16/1065; A61M 16/1055; A61M 16/107;**
A61M 16/0066; A61M 16/0833; A61M 16/0875;
A61M 16/208; A61M 16/209; A61M 16/22;
A61M 2205/0238; A61M 2205/7509;
A61M 2205/7518; A61M 2207/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2021 US 202117643347**

(71) Applicant: **Ambu A/S**
**2750 Ballerup (DK)**

(72) Inventors:
• **WINKLER, Kevin Joseph**
**2750 Ballerup (DK)**

• **HRUSKA, Amy Lee McQueen**
**2750 Ballerup (DK)**
• **GRAHAM, Peter Michael**
**2750 Ballerup (DK)**
• **BULLITT, Brandon**
**2750 Ballerup (DK)**
• **HEAVIN, Kleve Edwin**
**2750 Ballerup (DK)**

(74) Representative: **Guardian
IP Consulting I/S
Diplomvej, Building 381
2800 Kgs. Lyngby (DK)**

(54) **MEDICAL AIRWAY DEVICE**

(57) The medical airway device includes a housing having at least one breathing circuit port and including an airway filter arranged to filter air flowing through the at least one breathing circuit port. The airway filter includes a layered filter including at least a first separate filter layer and a second separate filter layer being stacked on top of each other so that neighbouring separate filter layers abut each other. The layered filter has a first surface being laminated with a first scrim layer and a second surface being laminated with a second scrim layer. Each separate filter layer is a non-woven fiber layer.

FIG. 17

EP 4 194 041 A1

**Description**

FIELD OF THE DISCLOSURE

The present disclosure relates to a medical airway device including a housing, the housing having at least one breathing circuit port, and the housing including an airway filter arranged to filter air flowing through the at least one breathing circuit port.

BACKGROUND

Medical filters are used in patient airway management situations, e.g. in breathing circuits that connect a patient to a respirator or anesthesia machine. The filters are used to protect the patient from inhaling inspiratory air containing airborne contaminants that may harm the patient, e.g. airborne particulates or aerosols or pathogens of microbial or viral origin that may be airborne by being adhered to or contained in aerosols of droplets of water/liquids. In addition, the filters may protect the respirator or anesthesia machine from being contaminated with pathogens in the expiratory air from patients suffering from airborne infectious diseases.

Hepa filters in the form of pleated relatively rigid plates of textile or paper-like filter material are used, but they are relatively expensive. Electrostatically charged non-woven filters have proven to be efficient in catching particulates or aerosols and are commonly used in medical filters used in airway management, because they are also less expensive than hepa filters and generally takes up less space than pleated HEPA filters.

US 10,857,321 B2 discloses a filter device including a distal housing comprising a distal inner port and a distal outer port; a proximal housing comprising a proximal inner port and a proximal outer port. The proximal housing is sealingly affixed to the distal housing to form an inspiratory pathway between the distal inner port and the proximal inner port and to form an expiratory pathway between the distal outer port and the proximal outer port that is fluidly sealed from the inspiratory pathway. The inspiratory pathway is laterally adjacent the expiratory pathway. A first filter is arranged in the inspiratory pathway or in the expiratory pathway to filter gases flowing through the inspiratory pathway or the expiratory pathway.

The diameter of the filters and consequently of the filter housings of filter devices as disclosed above is a balance of having a filter surface as large as possible in order to reduce filter resistance and at the same time to reduce the dead space that is inside the filter housing as much as possible, since the dead space in the filter housing may affect ventilation of the patient negatively. In particular in some breathing circuit configurations, increased dead space in the filter housing may lower the volume of fresh air delivered to the patient in each respiration cycle, because more expiratory air may mix with the fresh air intake before it is inhaled. Therefore, an increase of the filter diameter of existing filter devices is typically unwanted. Consequently, in order to meet stricter requirements in terms of viral filtration efficiency (VFE), a solution may be to increase the thickness of the filter material. However, this may again typically lead to increased resistance which may affect respiration negatively.

US 6,858,057 B2 discloses a filter media comprising a synthetic microfiber polymer fine fiber web wherein the diameter of the fibers is between about 0.8 to about 1.5 microns.

WO 2018/017937 A1 relates generally to filter media and methods for filtering gas streams, and specifically, to filter media having efficiency stability and/or good performance characteristics such as low resistance.

SUMMARY OF DISCLOSED EMBODIMENTS

The object of the present disclosure is to provide a medical airway device having increased viral filtration efficiency without increasing dead space and with as little increase of filtration resistance as possible.

In view of this object, the airway filter includes a layered filter including at least a first separate filter layer and a second separate filter layer, the at least first and second separate filter layers are stacked on top of each other so that neighbouring separate filter layers abut each other, the layered filter has a first surface being laminated with a first scrim layer and a second surface being laminated with a second scrim layer, and each separate filter layer is a non-woven fiber layer.

In this way, by stacking at least a first and a second separate filter layer on top of each other, the airway filter surprisingly has a lower filter resistance than a corresponding single layer airway filter having the same total basis weight, i.e. weight per area, and having the same material composition and fiber diameter. For instance, an airway filter having two non-woven fiber layers, each having a basis weight of 150 grams per square meter, has a lower filter resistance than a corresponding airway filter having a single non-woven fiber layer with a basis weight of 300 grams per square meter. The viral filtration efficiency of the layered filter is not significantly affected in relation to that of the comparable single layer filter. Thereby, the viral filtration efficiency may be increased substantially without a substantial increase in filter resistance.

In an embodiment, each separate filter layer is electrostatically-charged. Electrostatically charged fibers may contact and catch more aerosol droplets/particulates than fibers not being electrostatically charged, and thereby, filtration efficiency may be improved. Most particles and aerosols, and especially those of water, have a surface charge. Therefore, the electrostatically charged surface on the fibers in the filter may attract the charged surface on the aerosols/particles and thereby cause improved removal/filter efficiency.

In an embodiment, each separate filter layer is made of blended synthetic fibers.

In an embodiment, each separate filter layer comprises or consists of spun polypropylene fibers.

In an embodiment, each separate filter layer is a needle punched material.

In an embodiment, each separate filter layer is made of the same type of material, i.e. material composed by the same type of fibers and material having the same density.

In an embodiment, the first and second scrim layers are textile membranes of non-woven polypropylene. The textile membranes may be hydrophobic. The scrim layers may mitigate fiber migration from the non-woven fiber layers of the layered filter itself, which may be looser spunbond sheets than the scrim layers.

In an embodiment, the first and second scrim layers are needle punched onto their respective separate filter layers.

In an embodiment, the total basis weight of the layered filter including the separate filter layers, but excluding the scrim layers, is at least 250 grams per square meter.

In an embodiment, the basis weight of each separate filter layer is at least 20 per cent of the total basis weight of the layered filter including the separate filter layers, but excluding the scrim layers.

In an embodiment, the medical airway device is a filter device, the housing includes a proximal housing part and a distal housing part, the proximal housing part has a proximal connector forming a first breathing circuit port, the distal housing part has a distal connector forming a second breathing circuit port, the proximal housing part and the distal housing part are sealingly connected to each other, and the airway filter is arranged at the connection between the proximal housing part and the distal housing part.

In an embodiment, a foam sponge is arranged in the proximal housing part to filter air flowing between the single proximal connector and the airway filter.

In an embodiment, the medical airway device is a filter device, the housing includes a proximal housing part and a distal housing part, the proximal housing part has a proximal inspiratory connector and a proximal expiratory connector, each forming respective breathing circuit ports, the distal housing part has a distal inspiratory connector and a distal expiratory connector, each forming respective breathing circuit ports, the proximal housing part and the distal housing part are sealingly connected to each other, the airway filter is arranged at the connection between the proximal housing part and the distal housing part, the airway filter includes an inspiratory airway filter arranged to filter air flowing between the proximal inspiratory connector and the distal inspiratory connector, and the airway filter includes an expiratory airway filter arranged to filter air flowing between the proximal expiratory connector and the distal expiratory connector.

In an embodiment, the inspiratory airway filter and the expiratory airway filter are formed by separate parts of one single layered filter arranged in the housing.

In an embodiment, at least the distal inspiratory connector and the distal expiratory connector are arranged concentrically.

In an embodiment, at least the proximal inspiratory connector and the proximal expiratory connector are arranged non-concentrically, and one of the proximal inspiratory connector and the proximal expiratory connector extends laterally from the proximal housing part.

In an embodiment, the medical airway device is a resuscitator including a self-inflating squeeze bag having a squeeze bag inlet opening forming a first breathing circuit port and a squeeze bag outlet opening forming a second breathing circuit port, the squeeze bag inlet opening accommodates a squeeze bag inlet valve housing including a squeeze bag inlet valve arrangement being adapted to allow inflow of air into the squeeze bag and being adapted to prevent outflow of air from the squeeze bag through the squeeze bag inlet opening, the squeeze bag outlet opening accommodates a patient valve housing including a patient valve arrangement being adapted to allow outflow of air from the squeeze bag into the patient valve housing and being adapted to prevent inflow of air into the squeeze bag through the squeeze bag outlet opening, the patient valve housing further includes a patient connection port for ventilation of a patient and a patient expiration outlet port for outlet of exhaled gas from the patient valve housing to the surroundings, and the airway filter is located upstream the squeeze bag outlet opening in order to filter air before reaching the patient valve arrangement from the squeeze bag.

In an embodiment, the medical airway device is a face mask for ventilation and/or preoxygenation of a patient, the face mask includes a dome and a face contact cuff, the dome is provided with a connector forming the at least one breathing circuit port, and the airway filter is arranged inside the dome.

In an embodiment, the medical airway device is a PEEP valve for the control of flow and/or pressure at an expiratory connector forming the at least one breathing circuit port.

In an embodiment, the PEEP valve includes a PEEP valve housing having a peripheral PEEP valve housing wall, the peripheral PEEP valve housing wall is provided with a number of patient expiration outlet openings for outlet of exhaled gas from the PEEP valve housing to the surroundings, and the airway filter is arranged to filter air flowing through the patient expiration outlet openings.

In an embodiment, the airway filter is generally tubular and at least partly conical and/or partly cylindrical and covers an outside of the peripheral PEEP valve housing wall.

BRIEF DESCRIPTION OF THE DRAWING

The disclosure will now be explained in more detail below by means of examples of embodiments with reference to the very schematic drawing, in which

Fig. 1 illustrates an example of a dual limb breathing circuit configuration;

Fig. 2 illustrates an example of a single limb breathing circuit configuration;

Fig. 3 is a perspective view of an embodiment of a filter device having a single proximal connector and a single distal connector, the filter device including a foam sponge;

Fig. 4 is a longitudinal sectional view through the filter device of Fig. 3;

Fig. 5 is an exploded longitudinal sectional view through the filter device of Fig. 3;

Fig. 6 is a perspective view of another embodiment of a filter device having a single proximal connector and a single distal connector;

Fig. 7 is an exploded longitudinal sectional view through the filter device of Fig. 6;

Fig. 8 is a perspective view of an embodiment of a filter device having two concentric proximal connectors and two concentric distal connectors;

Fig. 9 is an exploded longitudinal sectional view through the filter device of Fig. 8;

Fig. 10 is a longitudinal sectional view through another embodiment of a filter device having two concentric proximal connectors and two concentric distal connectors;

Fig. 11 is a longitudinal sectional view through an embodiment of a filter device having two separate proximal connectors and two concentric distal connectors;

Fig. 12 illustrates, seen in an axial section, an embodiment of a medical airway device in the form of a resuscitator with an airway filter;

Fig. 13 illustrates, seen in an axial section, another embodiment of a medical airway device in the form of a resuscitator with an airway filter;

Fig. 14 is a perspective view of a medical airway device in the form of a face mask including an airway filter;

Fig. 15 is an axial section through the medical airway device of Fig. 14;

Fig. 16 is an axial section through a medical airway device in the form of a PEEP valve including an airway filter;

Fig. 17 is an exploded perspective view of an embodiment of an airway filter according to the present disclosure; and

Fig. 18 is a table illustrating comparative test results for different embodiments of airway filters.

DETAILED DESCRIPTION OF DISCLOSED EMBODIMENTS

In the following, generally, similar elements of different embodiments have been designated by the same reference numerals.

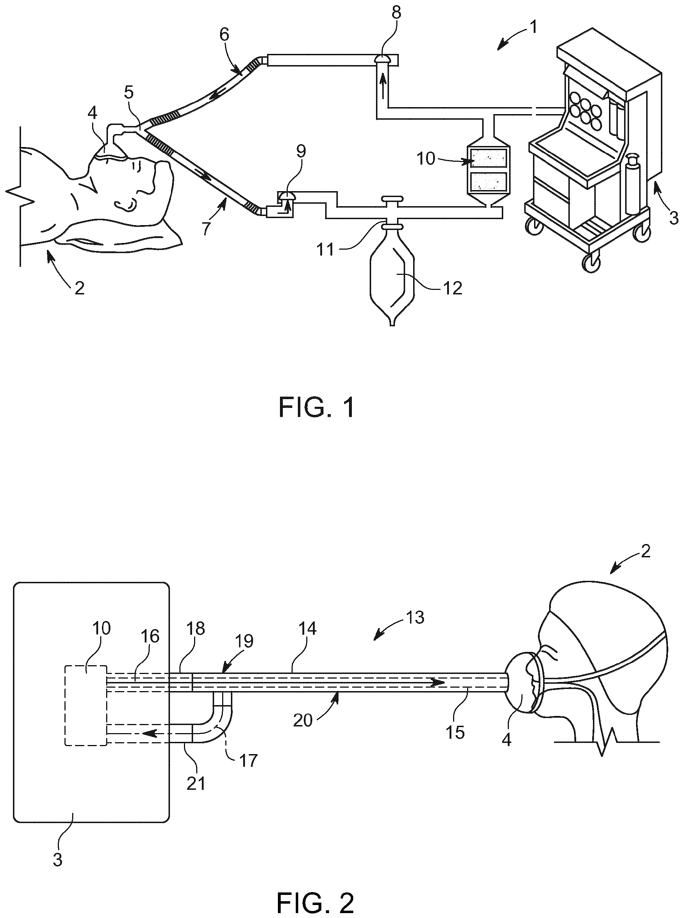

Fig. 1 illustrates a dual limb breathing circuit 1 in which a medical airway device according to the present disclosure may be incorporated as further explained below. The breathing circuit 1 connects a patient 2 to a respirator or anesthesia machine 3. A face mask 4 through which the patient 2 is ventilated is by means of a Y-piece 5 connected to an inspiratory limb 6 and an expiratory limb 7. The face mask 4 is just an example of a patient interface. Other examples of known patient interfaces are laryngeal tubes, laryngeal masks, endoctracheal tubes (single or dual lumen) or tracheostomy tubes (establishing an airway surgically). The inspiratory limb 6 is by means of a unidirectional valve 8 connected to the anesthesia machine 3 to receive fresh gas. The expiratory limb 7 is by means of a unidirectional valve 9 and a carbon dioxide absorber 10 connected to a fresh gas intake end of the inspiratory limb 6. Furthermore, the expiratory limb 7 is by means of an adjustable pressure-limiting (APL) valve 11 connected to a reservoir bag 12.

Fig. 2 illustrates a single limb breathing circuit 13 in which a medical airway device according to the present disclosure may be incorporated as further explained below. A unilimb breathing tube 20 of the single limb breathing circuit includes two coaxial tubes 14, 15, wherein the inner tube 15 delivers inspiratory air from the respirator/anesthesia machine 3 to the patient 2 and the outer tube 14 transports expiratory air from the patient to the respirator/anesthesia machine 3. The respirator/anesthesia machine 3 may include a carbon dioxide absorber 10 intermediate an inspiratory gas pathway 16 and an expiratory gas pathway 17. Fresh and recycled gases are supplied via inspiratory gas pathway 16 to the lungs of the patient. Expired gases pass through expiratory gas pathway 17 to return to respirator/anesthesia machine 3, where they may be scrubbed (in the $CO_2$ absorber 10) and recycled. Inspiratory gas pathway 16 includes pathways through an inspiratory gas outlet port 18, a manifold 19, and the inner tube 15 of the unilimb breathing tube 20. Expiratory gas pathway 17 includes pathways through the outer tube 14 of the unilimb breathing tube 20, the manifold 19, and an expiratory gas inlet port 21. Space at the distal end of the unilimb breathing tube 20 and within a face mask 4, where inspired gases and expired gases are not separated and can mix, is referred to as "deadspace". Additional components may also be included to connect unilimb breathing tube 20 and face mask 4, such as elbows and swivel connectors. These devices may change the orientation of the face mask 4 relative to the unilimb breathing tube 20.

According to the present disclosure, medical filters may be incorporated by means of a medical airway device in different ways both in dual limb breathing circuits 1 and in single limb breathing circuits 13 as described above in order to protect the patient from inhaling inspiratory air containing airborne contaminants that may harm the patient, e.g. airborne particulates or aerosols or pathogens of microbial or viral origin that may be airborne by being adhered to or contained in aerosols of droplets of water/liquids. In addition, the filters may protect the respirator or anesthesia machine 3 from being contaminated with pathogens in the expiratory air from patients suffering from airborne infectious diseases.

According to the present disclosure, a medical airway device includes an airway filter 32, 53, 54 having increased viral filtration efficiency without increasing dead space and without significantly affecting filtration resistance.

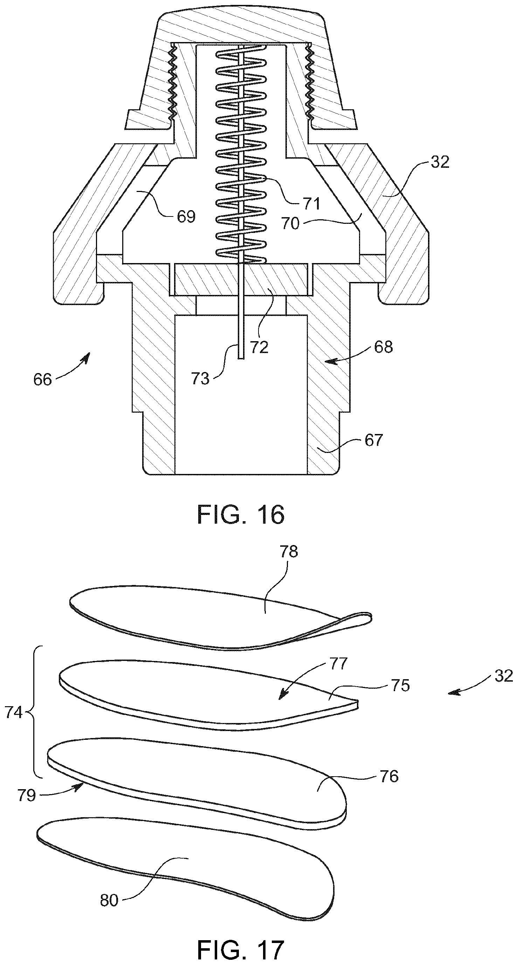

As illustrated in Fig. 17, the airway filter 32, 53, 54 includes a layered filter 74 including at least a first separate filter layer 75 and a second separate filter layer 76. The at least first and second separate filter layers 75, 76 are stacked on top of each other so that neighbouring separate filter layers abut each other. The layered filter 74 has a first surface 77 being laminated with a first scrim layer 78 and a second surface 79 being laminated with a second scrim layer 80. Each separate filter layer 75, 76 is a non-woven fiber layer. It may be preferred that the layered filter 74 includes exactly two separate filter layers 75, 76, but any suitable number of separate filter layers is possible. After stamping the dual layered filter into a particular shape suited for the application, such as for instance a circular filter, the filters may be arranged in a filter housing as further described below.

Each separate filter 75, 76 layer may preferably be electrostatically-charged, is preferably made of blended synthetic fibers, or is preferably made of or comprises spun polypropylene fibers. The filter layers 75, 76 may be non-woven mats of felts which means that they have the fibers in a random orientation (being neither woven or knitted). The fibers in non-woven felts are typically meltblown. Each separate filter layer may be a needle punched material and is preferably made of the same type of material when it comes to fiber material, fiber type, average fiber diameter, average fiber length, and fiber arrangement. The first and second scrim layers 78, 80 are preferably textile membranes of non-woven polypropylene and are preferably needle punched onto their respective separate filter layers. The non-woven scrim is spunbound which includes that the fibers are bonded thermally or by a resin, which gives a stronger textile. The total basis weight of the layered filter 74 including the separate filter layers 75, 76, but excluding the scrim layers 78, 80, is preferably at least 200 grams per square meter, more preferred at least 250 grams per square meter, and more preferred about 300 grams per square meter. The basis weight of each separate filter layer 75, 76 is at least 20 per cent, preferably at least 30 per cent, more preferred at least 40 per cent, and most preferred at least 45 per cent of the total basis weight of the layered filter including the separate filter layers 75, 76, but excluding the scrim layers 78, 80.

The at least two separate filter layers 75, 76 may include a filter material based on textiles. The separate filter layers 75, 76 may generally have the form of bacterial and/or viral filters. The layered filter 74 may be able to remove up to 99,99% or 99,9999% of virus or bacteria from air.

The layered filter 74 may include multiple layers of non-woven textiles made from fibres of polyester, polypropylene, acrylics or polystyrene or mixtures thereof. Alternatively, two, three or more layers (e.g. of different arrangements) of the same fibre or fibre mixture may be used. Each layer may be of different fibres and/or different arrangements. The layered filter 74 may include at least two or three layers of non-woven polypropylene fibres or acrylic/polystyrene non-woven fibres.

The layered filter 74 includes at least two non-woven fiber layers 75, 76 with a scrim layer 78, 80 on each surface. The layered filter 74 may be a flat or shaped filter with a non-woven fiber layer (e.g. of spun polymer fibers, such as a blend of different synthetic polymer fibers, or one type of fibers, such as spun polypropylene (PP) fibers). The fibers may be treated to have an electrostatic charged surface. The density of this non-woven filter layers is e.g. between 100 g/m2 and 400 g/m2 (Grams Per Square Meter). The density of each non-woven filter layer is preferably 150-350, more preferred 150-300 even more preferred 150-250 and most preferred 100-200 g/m2. The layered filter may be provided with scrim layers, such as thin (15g/m2) layers of hydrophobic textile membrane (of non-woven polymer, e.g. a polypropylene (PP)) on both surfaces of the layered filter.

The electrostatic charge on the surface of the fibers attract aerosols or particles that may carry microbes or virus. Most airborne bacteria and virus use aerosols as a transport media. The aerosols are produced when people exhale, speak, sneeze or cough. The surface charge of the aerosols results in that the aerosols are attracted to the electrostatically charged filter material, thus providing an improved filter efficiency. An example of suitable commercially available electrostatic filters are e.g. TECHNOSTAT® or TECHNOSTAT PLUS™ by Hollingsworth & Vose.

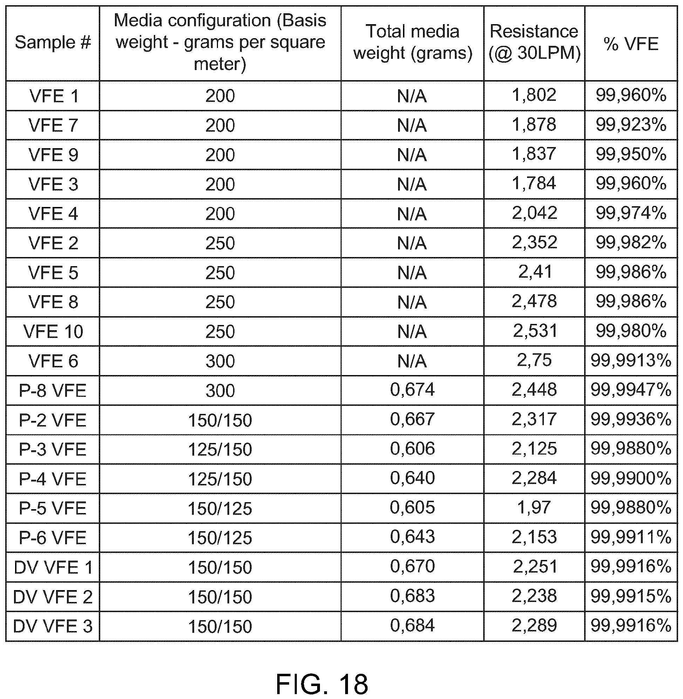

Fig. 18 illustrates various comparative test results for conventional single layer filters and layered filters having two separate filter layers stacked on top of each other according to the present disclosure, respectively. It is noted that LPM denotes litres per minute and that VFE denotes Viral Filtration Efficiency. Furthermore, the basis weight of Fig. 18 denotes the basis weight of the filter without scrim layers. In particular, it is noted that P-8 VFE is an airway filter having a single non-woven fiber layer with a basis weight of 300 grams per square meter and P-2 VFE is an airway filter having two non-woven fiber layers, each having a basis weight of 150 grams per square meter, thereby adding up to a total of 300 grams per square meter. As such, each of those two filters has the same total basis weight. As seen, the airway filter P-2 VFE having two non-woven fiber layers, each having a basis weight of 150 grams per square meter, has a lower filter resistance than the corresponding airway filter P-8 VFE having a single non-woven fiber layer with a basis weight of 300 grams per square meter. The viral filtration efficiency of the layered filter is only slightly lower than that of the comparable single layer filter. Thereby, the viral filtration efficiency may be increased substantially without a substantial increase in filter resistance.

Although not fully understood, the reason may be that because a 300 g/m2 filter is more dense and has smaller voids between the individual fibers in the non-woven layer compared with a 150g/m2 filter layer, the larger voids between the individual fibers may reduce the filter resistance and also allow the air to follow a labyrinthic or tortuous path through the filter media and thereby increase the chance of the possibly electrostatically charged fibers to contact and catch more aerosol droplets and/or particulates. Further, the intermediate space between the two discrete layers of non woven filter material may contribute in distributing air over the filter surface and/or may contribute to lowering the filter resistance.

It may further be concluded that, by stacking at least a first and a second separate filter layer on top of each other, the airway filter surprisingly has a lower filter resistance than a corresponding single layer airway filter having the same total basis weight, i.e. weight per area.

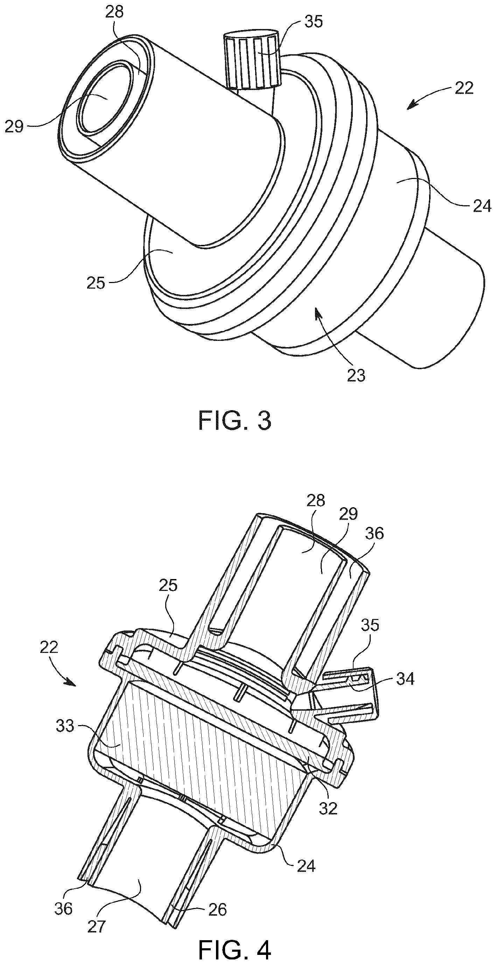

Figs. 3 to 5 illustrate a first embodiment of a medical airway device in the form of a filter device 22 according to the present disclosure. The filter device 22 has a housing 23 including a proximal housing part 25 and a distal housing part 24. The proximal housing part 25 has a single proximal connector 28 forming a first breathing circuit port 29, and the distal housing part 24 has a single distal connector 26 forming a second breathing circuit port 27. The proximal housing part 25 and the distal housing part 24 are sealingly connected to each other, for instance by being glued or ultrasonically welded along peripheral contact surfaces 30, 31. As seen, the peripheral contact surfaces 30, 31 may form a groove and tongue joint. An airway filter 32 is arranged at the connection between the proximal housing part 25 and the distal housing part 24. As seen, the airway filter 32 is flat and circular, and preferably, as seen in Fig. 4, the airway filter 32 is pinched between corresponding peripheral faces of the proximal housing part 25 and the distal housing part 24 in order to prevent a leak path around, instead of through, the airway filter 32. Furthermore, the distal housing part 24 is provided with a cylindrical moisture capturing foam sponge 33 arranged between the airway filter 32 and the second breathing circuit port 27. The foam sponge 33 is arranged for retaining moisture from and returning to the patient to help keep them from "drying out" during longer cases. It also helps prevent build-up of moisture in the breathing circuit itself. The proximal housing part 25 is optionally provided with an additional connection 34 provided with a detachable cap 35. The additional connection 34 may be used for gas sampling. It may be preferred to sample from the connection 34 because it means the air coming from the patient will be filtered before going to the gas analyser. As seen, both the proximal connector 28 and the distal connector 26 are provided with concentric walls forming a tubular insertion space 36 there between for the insertion of a tube connector in a well-known manner. The filter device 22 illustrated in Figs. 3 to 5 may for instance be used in the expiratory limb 7 of a dual limb breathing circuit as illustrated in Fig. 1, preferably at the distal/patient end of the breathing circuit. The filter device 22 may also be arranged between a resuscitator including a self-inflating squeeze bag and a corresponding face mask, laryngeal tube, laryngeal mask, endoctracheal tube (single or dual lumen), tracheostomy tube, etc.

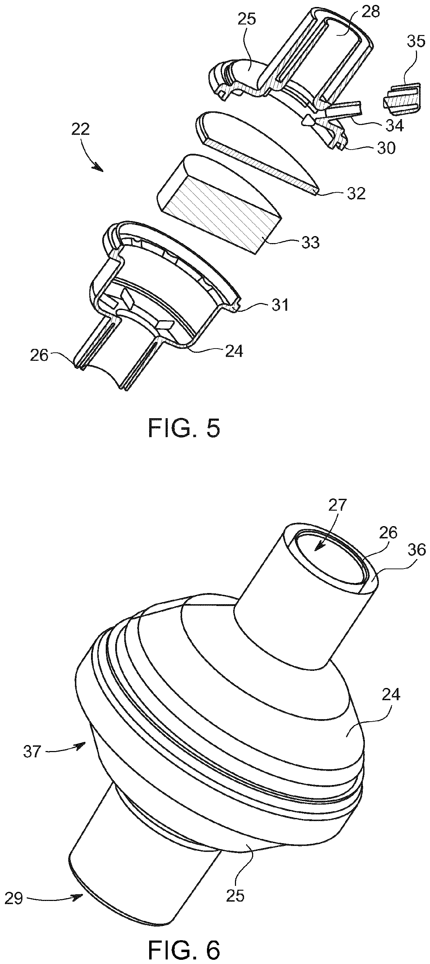

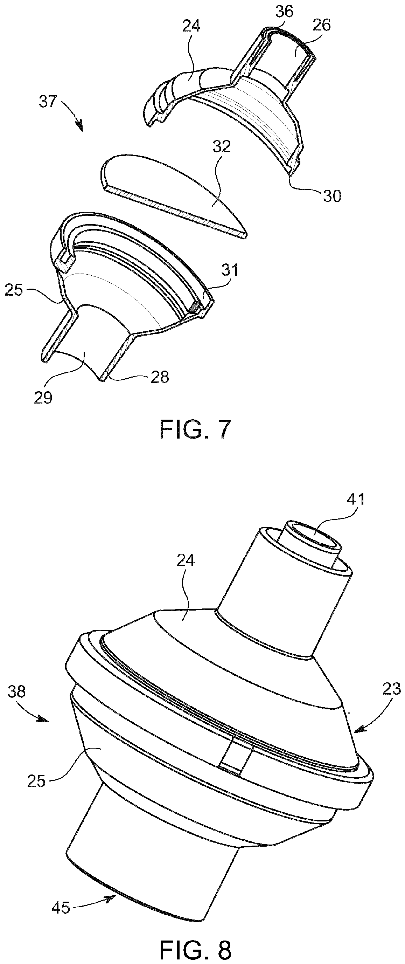

Figs. 6 and 7 illustrate a second embodiment of a medical airway device in the form of a filter device 37 according to the present disclosure. The filter device 22 has a housing 23 including a proximal housing part 25 and a distal housing part 24. The proximal housing part 25 has a single proximal connector 28 forming a first breathing circuit port 29, and the distal housing part 24 has a single distal connector 26 forming a second breathing circuit port 27. The filter device 23 differs from the filter device 22 of Figs. 3 to 5 mainly by not being provided with a moisture capturing foam sponge and not having an additional connection. Furthermore, it is noted that only the distal connector 26 is provided with concentric walls forming a tubular insertion space 36 there between for the insertion of a tube connector. The proximal connector 28 is provided with a single tubular wall for connection with a tube connector. In general, these connectors are standard connectors within the industry. The filter device 37 illustrated in Figs. 6 and 7 may for instance be used in the expiratory limb 7 or in the inspiratory limb 6 of a dual limb breathing circuit as illustrated in Fig. 1. The filter device 37 may also be arranged between a resuscitator including a self-inflating squeeze bag and a corresponding face mask.

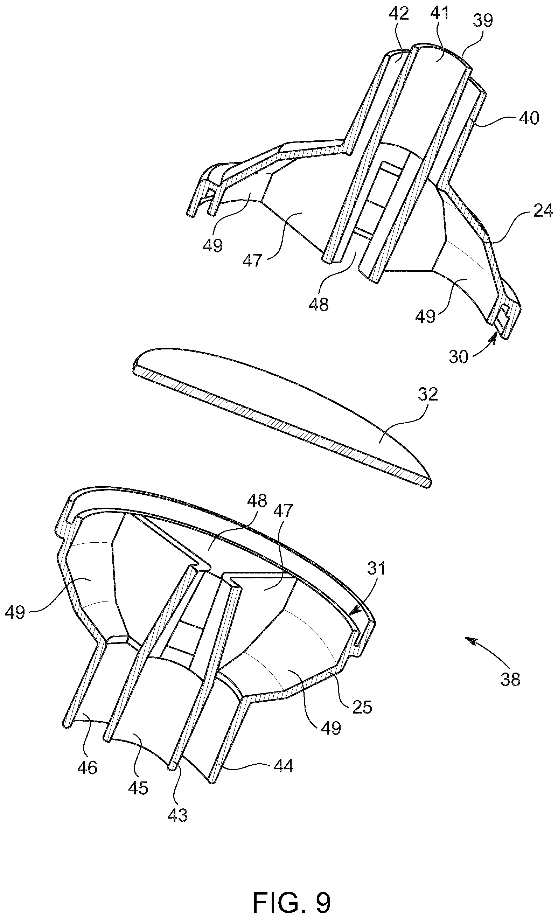

Figs. 8 and 9 illustrate a third embodiment of a medical airway device in the form of a filter device 38 according to the present disclosure. The filter device 38 has a housing 23 including a proximal housing part 25 and a distal housing part 24. The proximal housing part 25 has a proximal inspiratory connector 43 and a proximal expiratory connector 44 being arranged concentrically, and each forming respective breathing circuit ports 45, 46. The distal housing part 24 has a distal inspiratory connector 39 and a distal expiratory connector 40 being arranged concentrically, and each forming respective breathing circuit ports 41, 42. As seen, the proximal housing part 25 and the distal housing part 24 are both provided with internal separating walls 47 separating the internal part of the housing into one inspiratory channel 48 connecting the proximal inspiratory breathing circuit port 45 with the distal inspiratory breathing circuit port 41 and two distinct expiratory channels 49 both connecting the proximal expiratory breathing circuit port 46 with the distal expiratory breathing circuit port 42.

The proximal housing part 25 and the distal housing part 24 are sealingly connected to each other, for instance by being glued or ultrasonically welded along peripheral contact surfaces 30, 31. As seen, the peripheral contact surfaces 30, 31 may form a groove and tongue joint. An airway filter 32 is arranged at the connection between the proximal housing part 25 and the distal housing part 24. As seen, the airway filter 32 is flat and circular, and preferably, the airway filter 32 is pinched between corresponding peripheral faces of the proximal housing part 25 and the distal housing part 24 in order to prevent a leak path around, instead of through, the airway filter 32. Furthermore, the airway filter 32 is pinched between corresponding internal separating walls 47 of the proximal housing part 25 and the distal housing part 24, respectively, in order to prevent a leak path between the inspiratory channel 48 and the expiratory channels 49 of the housing 23. As seen, according to this third embodiment, the flat and circular airway filter 32 forms both an inspiratory airway filter arranged to filter air flowing between the proximal inspiratory connector 43 and the distal inspiratory connector 39 and an expiratory airway filter arranged to filter air flowing between the proximal expiratory connector 44 and the distal expiratory connector 40.

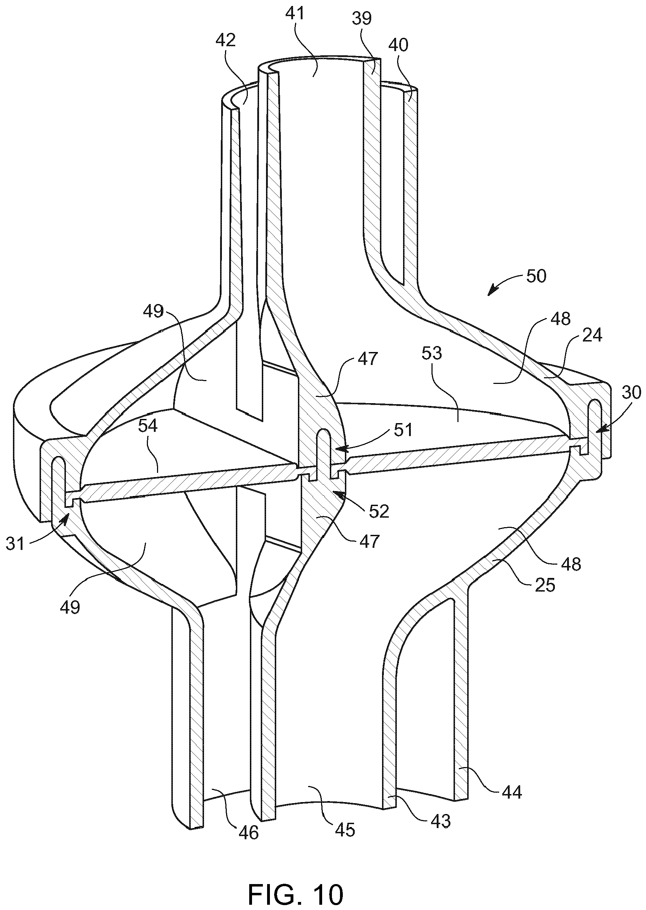

Fig. 10 illustrates a fourth embodiment of a medical airway device in the form of a filter device 50 according to the present disclosure. The filter device 50 corresponds to the filter device 38 illustrated in Figs. 8 and 9 apart from the following features. Whereas the internal part of the housing 23 of the filter device 38 is separated by means of internal separating walls 47 into one inspiratory channel 48 and two distinct expiratory channels 49, the internal part of the housing 23 of the filter device 50 is separated by means of internal separating walls 47 into one single inspiratory channel 48 and one single expiratory channel 49. Correspondingly, as seen in Fig. 10, the proximal housing part 25 and the distal housing part 24 of the filter device 50 are both provided with one single internal separating wall 47. Furthermore, as seen, corresponding contact parts 51, 52 of the respective separating walls 47 form a groove and tongue joint, thereby forming an airtight connection. In addition, as seen, the airway filter 32 is formed by means of one distinct inspiratory airway filter 53 and one distinct expiratory airway filter 54 arranged in the respective inspiratory channel 48 and expiratory channel 49 in that the inspiratory and expiratory airway filters 53, 54 are pinched between the contact parts 51, 52 of the respective separating walls 47 in addition to being pinched between the peripheral contact surfaces 30, 31 of the proximal housing part 25 and the distal housing part 24. The filter device 50 illustrated in Fig. 10 may for instance be arranged in the unilimb breathing tube 20 of the single limb breathing circuit 13 illustrated in Fig. 2.

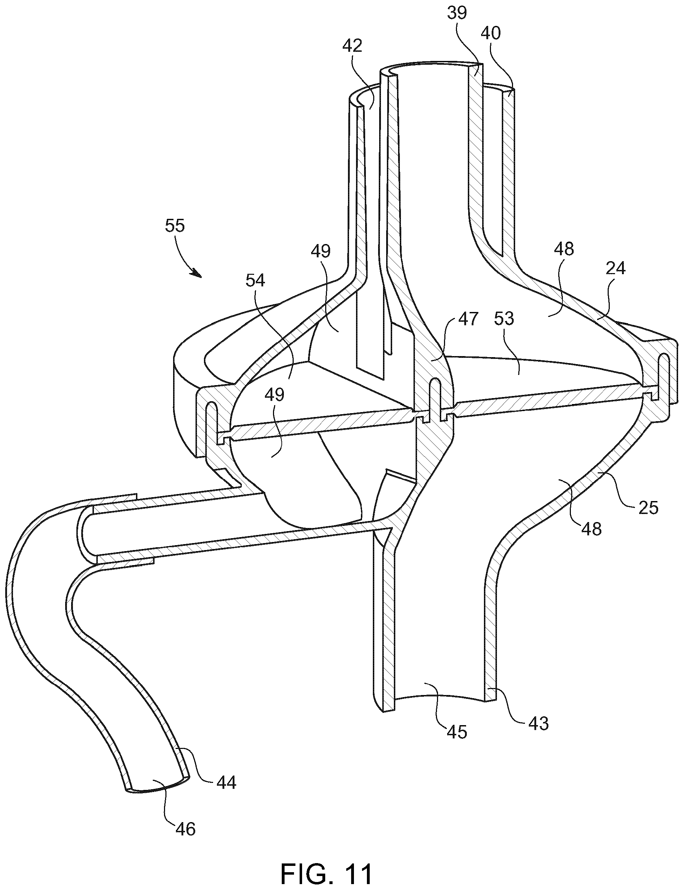

Fig. 11 illustrates a fifth embodiment of a medical airway device in the form of a filter device 55 according to the present disclosure. The filter device 55 corresponds to the filter device 50 illustrated in Fig. 10 apart from the following feature. As seen, the proximal inspiratory breathing circuit port 45 and the proximal expiratory breathing circuit port 46 are formed as separate tubes rather than being concentric. Thereby, the filter device 55 of this fifth embodiment could form the manifold 19 of the single limb breathing circuit 13 illustrated in Fig. 2.

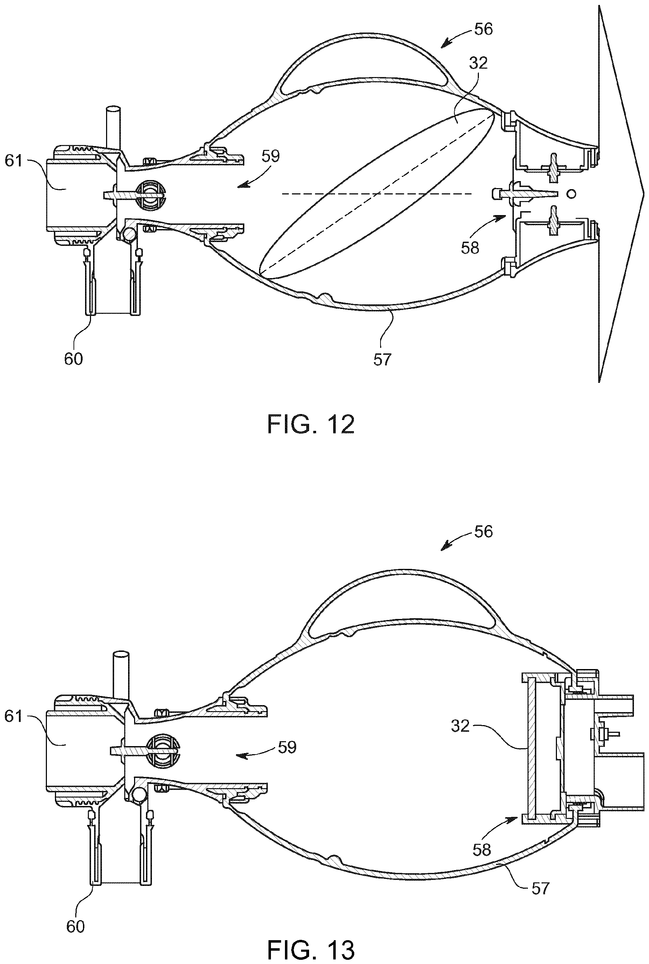

Fig. 12 illustrates a sixth embodiment of a medical airway device in the form of a resuscitator 56 including a self-inflating squeeze bag 57 having a squeeze bag inlet opening 58 forming a first breathing circuit port and a squeeze bag outlet opening 59 forming a second breathing circuit port. The squeeze bag inlet opening 58 accommodates a squeeze bag inlet valve housing 59 including a squeeze bag inlet valve arrangement being adapted to allow inflow of air into the squeeze bag and being adapted to prevent outflow of air from the squeeze bag through the squeeze bag inlet opening 58. The squeeze bag outlet opening 59 accommodates a patient valve housing including a patient valve arrangement being adapted to allow outflow of air from the squeeze bag 57 into the patient valve housing and being adapted to prevent inflow of air into the squeeze bag through the squeeze bag outlet opening 59. The patient valve housing further includes a patient connection port 60 for ventilation of a patient and a patient expiration outlet port 61 for outlet of exhaled gas from the patient valve housing to the surroundings. The airway filter 32 is located upstream the squeeze bag outlet opening 59 in order to filter air before reaching the patient valve arrangement from the squeeze bag 57. As further seen, in the embodiment illustrated in Fig. 12, the squeeze bag 57 is elongated in a longitudinal direction extending from the squeeze bag inlet opening 58 to the squeeze bag outlet opening 59, and the airway filter 32 has a longitudinal direction being arranged obliquely to the longitudinal direction of the squeeze bag 57. Thereby, the filter area of the airway filter 32 may be maximized.

The airway filter 32 can also be arranged at the transit between the squeeze bag 57 and the patient valve housing. The shown positions of the airway filter 32 in or on the resuscitator are merely exemplary and non limiting since it is clear that the airway filter 32 could be placed anywhere upstreams to the patient valve, including upstreams of the inlet valve housing.

International Patent Application No. PCT/EP2021/077256 disclosing a resuscitator including a self-inflating squeeze bag is hereby incorporated by reference. Some of the filters in the disclosed resuscitator are not flat, but could e.g. be made from a flat sheet of the dual layered filter with a welding seam along the length/height of a cylinder/cone. For instance the filter could have a welding seam along the length of the cylinder that is similar to the welding seam along the outer circumference of the circular flat filter disclosed for filter devices in the present disclosure.

Fig. 13 illustrates a seventh embodiment of a medical airway device in the form of a resuscitator 56 corresponding to that of Fig. 12. However, in the embodiment of Fig. 13, the airway filter 32 is arranged as a flat circular filter covering and being attached at the squeeze bag inlet opening 58.

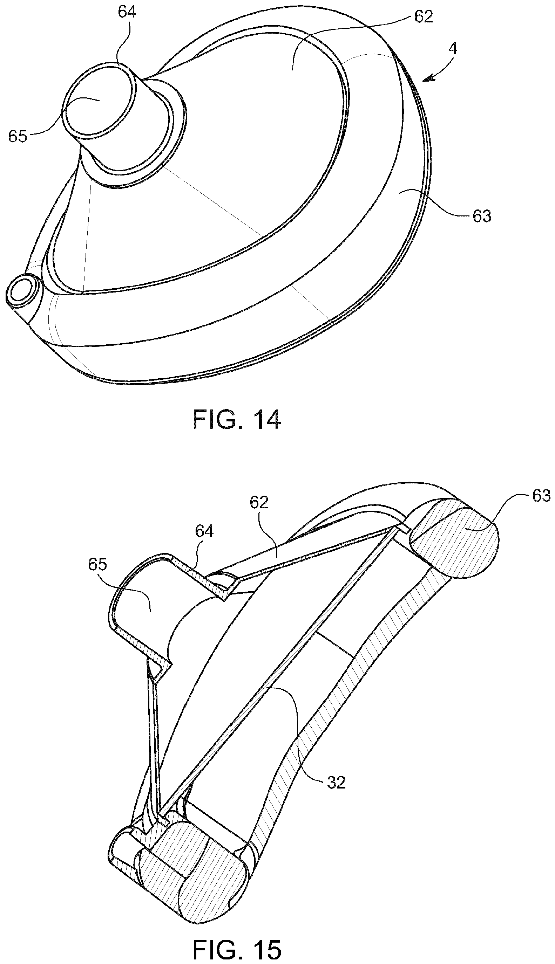

Figs. 14 and 15 illustrate an eight embodiment of a medical airway device in the form of a face mask 4 for ventilation of a patient. The face mask 4 includes a dome 62 and a face contact cuff 63. The dome 62 is provided with a connector 64 forming a breathing circuit port 65, and the airway filter 32 is arranged inside the dome 62. The position of the airway filter 32 illustrated in the figure is merely exemplary as it could be placed at any preferred distance between the face contact cuff 63 and the connector 64. At least it could be positioned so that it would not touch the patient's nose or any other part of the face. The face mask 4 may be used as illustrated in Figs. 1 and 2.

Fig. 16 illustrates a ninth embodiment of a medical airway device in the form of a PEEP valve 66 for the control of flow and/or pressure at an expiratory connector 67 forming a breathing circuit port. The PEEP valve 66 includes a PEEP valve housing 68 having a peripheral PEEP valve housing wall 69. The peripheral PEEP valve housing wall 69 is provided with a number of patient expiration outlet openings 70 for outlet of exhaled gas from the PEEP valve housing 68 to the surroundings. The airway filter 32 is arranged to filter air flowing through the patient expiration outlet openings 70. In this illustrated embodiment, the airway filter 32 is generally tubular in that it is composed of a tubular conical part and a tubular cylindrical part and covers an outside of the peripheral PEEP valve housing wall 69.

International Patent Application No. PCT/EP2021/077256 mentioned above and incorporated by reference also disclosed various embodiments of PEEP valves. The cylindrical/conical filters in such PEEP valves as well as in the above described resuscitator could in principle also be made from a flat filter that is attached to the filter housing by squeezing the "seam" along the length/height of a cylinder/cone between two housing parts (inner outer housing parts), e.g. if a protecting "grille" is necessary. It would be possible to use the same principle(s) as described in US 10,857,321 B2 which is hereby incorporated by reference. These principle(s) are e.g. described in Figs. 9-10 of US 10,857,321 B2. US 2021/0069450 A1 describing similar solutions is equally incorporated by reference in this description.

It is also possible to attach flat filters to the valve housing in the resuscitator or PEEP valve described above by using the same principle as described and illustrated in Figs. 9-10 of US 10,857,321 B2 and in US 2021/0069450 A1, also along the length of the cone/cylinder. All that is needed is to cut the filters in a shape that results in the cylinder (i.e. a rectangular filter pad) or a conical filter when wrapped "around" the inner valve housing.

The cylindrically/conically shaped filters could also be made from flat filters that are "squeezed" along the length as well by adding a separate "squeezing" bar/frame that covers the edges of the filter which can then be glued or welded (ultrasonically) to the valve housing by using the same connection principle as described and illustrated in Figs. 9-10 of US 10,857,321 B2 and in US 2021/0069450 A1.

Example:

As mentioned above, Fig. 18 is a table illustrating comparative test results for different embodiments of airway filters.

The test procedure was performed to evaluate the viral filtration efficiency (VFE) of test articles at an increased challenge level. The test article was a single layer or dual layer filter pad with a filter media configuration as specified in column 2 in Fig. 18. arranged in a commercially available HMEF filter housing as shown in Figs. 3-5 and provided by Ambu Inc. (USA). A suspension of $\Phi$X174 bacteriophage was delivered to the test article at a challenge level of greater than 106 plaque-forming units (PFU) to determine the filtration efficiency. Challenge suspension level (C) was $8.7 \times 10^6$ PFU. The suspension was aerosolized using a nebulizer and delivered to the test article, i.e. the filter, at a fixed air pressure and flow rate of 30 liters per minute (LPM). The aerosol droplets were generated in a glass aerosol chamber and drawn through the test article into all glass impingers (AGIs) for collection. The challenge suspension was delivered for a one minute interval and sampling through the AGIs was conducted for two minutes to clear the aerosol chamber. The mean particle size (MPS) control was performed at a flow rate of 28.3 LPM using a sixstage, viable particle, Andersen sampler for collection. The VFE at an Increased Challenge Level test procedure was adapted from the bacterial filtration efficiency test method as described in ASTM F2101-19.

This test procedure was modified to provide a VFE test procedure in order to employ a more severe challenge than would be experienced in normal use. All test method acceptance criteria were met. Testing was performed in compliance with US FDA good manufacturing practice (GMP) regulations 21 CFR Parts 210, 211 and 820.

The filter resistance has been measured as the pressure drop at 30 l/min in accordance with ISO 9360-1 (2nd edition, 2009-06-10).

The filtration efficiency percentages were calculated using the following equation:

$$\% VFE = ((C\text{-}T)/C) \; x \; 100$$

C = Challenge Level
T = Total PFU recovered downstream of the test article

For instance, for P-2 VFE 150/150 of the table of Fig. 18, the total PFU recovered was measured to 5.6 x $10^2$, resulting in a filtration efficiency of 99.9936% calculated by using the above equation.

Table 1 % VFE test results of selected tests from Fig. 18.

| Test article no. ( media configuration cf. Fig 18) | Total PFU recovered | % VFE |
|---|---|---|
| P-2 VFE (150/150) | $5.6 \times 10^2$ | 99.9936 |
| P-3 VFE (125/150) | $1.0 \times 10^3$ | 99.988 |
| P-4 VFE (125/150) | $9.0 \times 10^2$ | 99.990 |
| P-5 VFE (150/125) | $1.0 \times 10^3$ | 99.988 |
| P-6 VFE (150/125) | $7.7 \times 10^2$ | 99.9911 |
| P-8 VFE (300) | $4.6 \times 10^2$ | 99.9947 |

LIST OF REFERENCE NUMBERS

[0067]

| | |
|---|---|
| 1 | dual limb breathing circuit |
| 2 | patient |
| 3 | respirator or anesthesia machine |
| 4 | face mask |
| 5 | Y-piece |
| 6 | inspiratory limb |
| 7 | expiratory limb |
| 8, 9 | unidirectional valve |
| 10 | carbon dioxide absorber |
| 11 | adjustable pressure-limiting (APL) valve |
| 12 | reservoir bag |
| 13 | single limb breathing circuit |
| 14 | outer coaxial tube |
| 15 | inner coaxial tube |
| 16 | inspiratory gas pathway |
| 17 | expiratory gas pathway |
| 18 | inspiratory gas outlet port |
| 19 | manifold |
| 20 | unilimb breathing tube |
| 21 | expiratory gas inlet port |

| | |
|---|---|
| 22 | filter device according to first embodiment |
| 23 | housing of filter device |
| 24 | distal housing part |
| 25 | proximal housing part |
| 26 | single distal connector |
| 27 | second breathing circuit port |
| 28 | single proximal connector |
| 29 | first breathing circuit port |
| 30, 31 | peripheral contact surface |
| 32 | airway filter |
| 33 | cylindrical moisture capturing foam sponge |
| 34 | additional connection |
| 35 | detachable cap |
| 36 | tubular insertion space |
| 37 | filter device according to second embodiment |
| 38 | filter device according to third embodiment |
| 39 | distal inspiratory connector |
| 40 | distal expiratory connector |
| 41 | distal inspiratory breathing circuit port |
| 42 | distal expiratory breathing circuit port |
| 43 | proximal inspiratory connector |
| 44 | proximal expiratory connector |
| 45 | proximal inspiratory breathing circuit port |
| 46 | proximal expiratory breathing circuit port |
| 47 | internal separating wall |
| 48 | inspiratory channel of housing |
| 49 | expiratory channel of housing |
| 50 | filter device according to fourth embodiment |
| 51, 52 | contact part of separating wall |
| 53 | inspiratory airway filter |
| 54 | expiratory airway filter |
| 55 | filter device according to fifth embodiment |
| 56 | resuscitator |
| 57 | self-inflating squeeze bag |
| 58 | squeeze bag inlet opening |
| 59 | squeeze bag outlet opening |
| 60 | patient connection port |
| 61 | patient expiration outlet port |
| 62 | dome |
| 63 | face contact cuff |
| 64 | connector |
| 65 | breathing circuit port |
| 66 | PEEP valve |
| 67 | expiratory connector |
| 68 | PEEP valve housing |
| 69 | PEEP valve housing wall |
| 70 | patient expiration outlet opening |
| 71 | spring of PEEP valve |
| 72 | displaceable valve element of PEEP valve |
| 73 | spindle of PEEP valve |
| 74 | layered filter |
| 75 | first separate filter layer |
| 76 | second separate filter layer |
| 77 | first surface of layered filter |
| 78 | first scrim layer |
| 79 | second surface of layered filter |
| 80 | second scrim layer |

**Claims**

1. A medical airway device including a housing, the housing having at least one breathing circuit port, the housing including an airway filter arranged to filter air flowing through the at least one breathing circuit port, the airway filter including a layered filter including at least a first separate filter layer and a second separate filter layer, the at least first and second separate filter layers being stacked on top of each other so that neighbouring separate filter layers abut each other, the layered filter having a first surface being laminated with a first scrim layer and a second surface being laminated with a second scrim layer, and each separate filter layer being a non-woven fiber layer.

2. The medical airway device of claim 1, wherein each separate filter layer is electrostatically-charged.

3. The medical airway device of claim 1 or 2, wherein each separate filter layer is made of blended synthetic fibers.

4. The medical airway device of anyone of the preceding claims, wherein each separate filter layer comprises or consists of spun polypropylene fibers.

5. The medical airway device of anyone of the preceding claims, wherein each separate filter layer is a needle punched material.

6. The medical airway device of anyone of the preceding claims, wherein each separate filter layer is made of the same material.

7. The medical airway device of anyone of the preceding claims, wherein the first and second scrim layers are textile membranes of non-woven polypropylene.

8. The medical airway device of anyone of the preceding claims, wherein the first and second scrim layers are needle punched onto their respective separate filter layers.

9. The medical airway device of anyone of the preceding claims, wherein the total basis weight of the layered filter including the separate filter layers, but excluding the scrim layers, is at least 240 grams per square meter.

10. The medical airway device of anyone of the preceding claims, wherein the basis weight of each separate filter layer is at least 20 per cent of the total basis weight of the layered filter including the separate filter layers, but excluding the scrim layers.

11. The medical airway device of anyone of the preceding claims, wherein at least the distal inspiratory connector and the distal expiratory connector are arranged concentrically.

12. The medical airway device of anyone of the preceding claims, wherein the medical airway device is a filter device, wherein the housing includes a proximal housing part and a distal housing part, wherein the proximal housing part has a proximal connector forming a first breathing circuit port, wherein the distal housing part has a distal connector forming a second breathing circuit port, wherein the proximal housing part and the distal housing part are sealingly connected to each other, and wherein the airway filter is arranged at the connection between the proximal housing part and the distal housing part.

13. The medical airway device of claim 12, wherein a foam sponge is arranged in the proximal housing part to filter air flowing between the single proximal connector and the airway filter.

14. The medical airway device of anyone of the preceding claims 1 to 11, wherein the medical airway device is a filter device, wherein the housing includes a proximal housing part and a distal housing part, wherein the proximal housing part has a proximal inspiratory connector and a proximal expiratory connector, each forming respective breathing circuit ports, wherein the distal housing part has a distal inspiratory connector and a distal expiratory connector, each forming respective breathing circuit ports, wherein the proximal housing part and the distal housing part are sealingly connected to each other, wherein the airway filter is arranged at the connection between the proximal housing part and the distal housing part, wherein the airway filter includes an inspiratory airway filter arranged to filter air flowing between the proximal inspiratory connector and the distal inspiratory connector, and wherein the airway filter includes an expiratory airway filter arranged to filter air flowing between the proximal expiratory connector and the distal expiratory connector.

**15.** The medical airway device of claim 14, wherein the inspiratory airway filter and the expiratory airway filter are formed by separate parts of one single layered filter arranged in the housing.

**16.** The medical airway device of claim 14, wherein at least the distal inspiratory connector and the distal expiratory connector are arranged concentrically.

**17.** The medical airway device of any one of claims 14 to 16, wherein at least the proximal inspiratory connector and the proximal expiratory connector are arranged non-concentrically, and wherein one of the proximal inspiratory connector and the proximal expiratory connector extends laterally from the proximal housing part.

**18.** The medical airway device of anyone of the preceding claims 1 to 10, wherein the medical airway device is a resuscitator including a self-inflating squeeze bag having a squeeze bag inlet opening forming a first breathing circuit port and a squeeze bag outlet opening forming a second breathing circuit port, wherein the squeeze bag inlet opening accommodates a squeeze bag inlet valve housing including a squeeze bag inlet valve arrangement being adapted to allow inflow of air into the squeeze bag and being adapted to prevent outflow of air from the squeeze bag through the squeeze bag inlet opening, wherein the squeeze bag outlet opening accommodates a patient valve housing including a patient valve arrangement being adapted to allow outflow of air from the squeeze bag into the patient valve housing and being adapted to prevent inflow of air into the squeeze bag through the squeeze bag outlet opening, wherein the patient valve housing further includes a patient connection port for ventilation of a patient and a patient expiration outlet port for outlet of exhaled gas from the patient valve housing to the surroundings, and wherein the airway filter is located upstream the squeeze bag outlet opening in order to filter air before reaching the patient valve arrangement from the squeeze bag.

**19.** The medical airway device of anyone of the preceding claims 1 to 10, wherein the medical airway device is a face mask for ventilation and/or preoxygenation of a patient, wherein the face mask includes a dome and a face contact cuff, wherein the dome is provided with a connector forming the at least one breathing circuit port, and wherein the airway filter is arranged inside the dome.

**20.** The medical airway device of anyone of the preceding claims 1 to 10, wherein the medical airway device is a PEEP valve for the control of flow and/or pressure at an expiratory connector forming the at least one breathing circuit port.

**21.** The medical airway device of claim 20, wherein the PEEP valve includes a PEEP valve housing having a peripheral PEEP valve housing wall, wherein the peripheral PEEP valve housing wall is provided with a number of patient expiration outlet openings for outlet of exhaled gas from the PEEP valve housing to the surroundings, and wherein the airway filter is arranged to filter air flowing through the patient expiration outlet openings.

**22.** The medical airway device of claim 19, wherein the airway filter is generally tubular and at least partly conical and/or partly cylindrical and covers an outside of the peripheral PEEP valve housing wall.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

| Sample # | Media configuration (Basis weight - grams per square meter) | Total media weight (grams) | Resistance (@ 30LPM) | % VFE |
|---|---|---|---|---|
| VFE 1 | 200 | N/A | 1,802 | 99,960% |
| VFE 7 | 200 | N/A | 1,878 | 99,923% |
| VFE 9 | 200 | N/A | 1,837 | 99,950% |
| VFE 3 | 200 | N/A | 1,784 | 99,960% |
| VFE 4 | 200 | N/A | 2,042 | 99,974% |
| VFE 2 | 250 | N/A | 2,352 | 99,982% |
| VFE 5 | 250 | N/A | 2,41 | 99,986% |
| VFE 8 | 250 | N/A | 2,478 | 99,986% |
| VFE 10 | 250 | N/A | 2,531 | 99,980% |
| VFE 6 | 300 | N/A | 2,75 | 99,9913% |
| P-8 VFE | 300 | 0,674 | 2,448 | 99,9947% |
| P-2 VFE | 150/150 | 0,667 | 2,317 | 99,9936% |
| P-3 VFE | 125/150 | 0,606 | 2,125 | 99,9880% |
| P-4 VFE | 125/150 | 0,640 | 2,284 | 99,9900% |
| P-5 VFE | 150/125 | 0,605 | 1,97 | 99,9880% |
| P-6 VFE | 150/125 | 0,643 | 2,153 | 99,9911% |
| DV VFE 1 | 150/150 | 0,670 | 2,251 | 99,9916% |
| DV VFE 2 | 150/150 | 0,683 | 2,238 | 99,9915% |
| DV VFE 3 | 150/150 | 0,684 | 2,289 | 99,9916% |

## FIG. 18

## EUROPEAN SEARCH REPORT

**Application Number**

EP 22 21 2076

### DOCUMENTS CONSIDERED TO BE RELEVANT

| Category | Citation of document with indication, where appropriate, of relevant passages | Relevant to claim |
|---|---|---|
| X | WO 2016/069342 A1 (3M INNOVATIVE PROPERTIES CO [US]) 6 May 2016 (2016-05-06) * figures 4,5 * * page 13, line 16 – page 20, line 27 * ----- | 1-22 |
| X | WO 01/32292 A1 (HOLLINGSWORTH & VOSE CO [US]) 10 May 2001 (2001-05-10) * figure 3 * * page 2, line 23 – page 24, line 2 * ----- | 1-22 |
| X | WO 2018/175550 A1 (HOLLINGSWORTH & VOSE CO [US]) 27 September 2018 (2018-09-27) * figures 6,7 * * page 9, line 10 – page 18, line 14 * * page 64, line 11 – page 64, line 26 * ----- | 1-22 |
| X | US 2017/232372 A1 (ZHANG XIAODAN [US] ET AL) 17 August 2017 (2017-08-17) * figures 1D, 1E, 1F, 1G * * paragraph [0027] – paragraph [0078] * * paragraph [0105] * * paragraph [0141] * ----- | 1-22 |
| X | WO 2016/100300 A2 (HOLLINGSWORTH & VOSE CO [US]) 23 June 2016 (2016-06-23) * figure 1 * * page 5, line 19 – page 13, line 22 * * page 45, line 6 – page 45, line 22 * * page 50, line 3 – page 50, line 9 * ----- | 1-22 |
| X | US 2021/121804 A1 (SMITHIES ALAN [US] ET AL) 29 April 2021 (2021-04-29) * figure 2 * * paragraph [0033] – paragraph [0047] * ----- | 1-22 |

-/--

**CLASSIFICATION OF THE APPLICATION (IPC)**

INV.
A61M16/10

ADD.
A61M16/20
A61M16/22
A61M16/08
A61M16/00

**TECHNICAL FIELDS SEARCHED (IPC)**

A61M

The present search report has been drawn up for all claims

| Place of search | Date of completion of the search | Examiner |
|---|---|---|
| Munich | 28 April 2023 | Liess, Helmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2022/078797 A1 (AMBU AS [DK]) 21 April 2022 (2022-04-21) * figure 17 * * page 22, line 27 – page 23, line 23 * ----- | 1-22 | |
| A | WO 2017/070696 A1 (KINGS SYSTEMS CORP [US]; WINKLER KEVIN [US] ET AL.) 27 April 2017 (2017-04-27) * the whole document * ----- | 1-22 | |

TECHNICAL FIELDS
SEARCHED        (IPC)

The present search report has been drawn up for all claims

| Place of search | Date of completion of the search | Examiner |
|---|---|---|
| Munich | 28 April 2023 | Liess, Helmar |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 4 194 041 A1**

## ANNEX TO THE EUROPEAN SEARCH REPORT
## ON EUROPEAN PATENT APPLICATION NO.

EP 22 21 2076

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2023

| Patent document cited in search report | Publication date | Patent family member(s) | Publication date |
|---|---|---|---|
| WO 2016069342 A1 | 06-05-2016 | BR 112017008761 A2 | 19-12-2017 |
| | | CA 2966012 A1 | 06-05-2016 |
| | | CN 107072341 A | 18-08-2017 |
| | | EP 3212018 A1 | 06-09-2017 |
| | | JP 2018500467 A | 11-01-2018 |
| | | KR 20170078717 A | 07-07-2017 |
| | | RU 2017114935 A | 04-12-2018 |
| | | SG 11201703267T A | 30-05-2017 |
| | | US 2017252590 A1 | 07-09-2017 |
| | | WO 2016069342 A1 | 06-05-2016 |
| WO 0132292 A1 | 10-05-2001 | AT 417660 T | 15-01-2009 |
| | | AU 1440801 A | 14-05-2001 |
| | | EP 1276548 A1 | 22-01-2003 |
| | | US 6554881 B1 | 29-04-2003 |
| | | US 2004035095 A1 | 26-02-2004 |
| | | WO 0132292 A1 | 10-05-2001 |
| WO 2018175550 A1 | 27-09-2018 | CN 110430932 A | 08-11-2019 |
| | | EP 3600603 A1 | 05-02-2020 |
| | | US 2018272258 A1 | 27-09-2018 |
| | | WO 2018175550 A1 | 27-09-2018 |
| US 2017232372 A1 | 17-08-2017 | US 2017232372 A1 | 17-08-2017 |
| | | US 2021236970 A1 | 05-08-2021 |
| WO 2016100300 A2 | 23-06-2016 | CN 106999818 A | 01-08-2017 |
| | | EP 3233239 A2 | 25-10-2017 |
| | | US 2016166953 A1 | 16-06-2016 |
| | | WO 2016100300 A2 | 23-06-2016 |
| US 2021121804 A1 | 29-04-2021 | NONE | |
| WO 2022078797 A1 | 21-04-2022 | NONE | |
| WO 2017070696 A1 | 27-04-2017 | EP 3365072 A1 | 29-08-2018 |
| | | US 2018311459 A1 | 01-11-2018 |
| | | US 2021069450 A1 | 11-03-2021 |
| | | WO 2017070696 A1 | 27-04-2017 |

EPO FORM P0459

For more details about this annex : see Official Journal of the European Patent Office, No. 12/82

## REFERENCES CITED IN THE DESCRIPTION

**Patent documents cited in the description**

- US 10857321 B2
- US 6858057 B2
- WO 2018017937 A1
- EP 2021077256 W
- US 20210069450 A1